# EUROPEAN PATENT SPECIFICATION

(11) **EP 0 641 771 B1**
(45) Date of publication and mention of the grant of the patent: **02.12.1998**
(21) Application number: 93307073.2
(22) Date of filing: 08.09.1993
(51) Int. Cl.: C07C 253/26

(54) **Method for ammoxidation of olefins**
Verfahren zur Ammoxydierung von Olefine
Procédé d'ammoxydation d'oléfines

(43) Date of publication of application: 08.03.1995
(73) Proprietor: THE STANDARD OIL COMPANY, Cleveland, Ohio 44114-2375 (US)
(72) Inventor: Friedrich, Maria S., Lyndhurst, Ohio 44124 (US); Seeley, Michael J., Twinsburg, Ohio 44022 (US); Paparizos, Christos, Willowick, Ohio 44094 (US); Suresh, Dev D., Hudson, Ohio 44236 (US)
(74) Representative: Crack, Richard David

(56) References cited:
- EP-A- 0 031 693
- BE-A- 766 843
- US-A- 3 681 421
- US-A- 5 008 427

## Description

This invention relates to a method for the catalytic vapor phase ammoxidation of C₃ to C₅ olefins to a,b-unsaturated mononitriles and HCN. In a more particular aspect the invention relates to a method for the catalytic vapor phase ammoxidation (1) of propylene to produce acrylonitrile and HCN and (2) isobutylene to produce methacrylonitrile and HCN. In the term "a,b-unsaturated mononitriles"_{,} "unsaturated" means ethylenic unsaturation, and the term does not include acetylenic unsaturation.

The present method employs an oxidic catalyst containing V, Sb and one or more of Sn, Ti, Fe and Ga promoters which increase the activity.

U.S. Patent 4,162,992 discloses the ammoxidation of olefins, especially propylene, using a catalyst having the formula

SbₐVTi_{c}Oₓ

where a is at least 6 and c is a number such that the ratio c/a is at least 0.5. As will be seen, however, the amount of Sb is far outside the range of the catalysts used in the presently claimed process.

In U.S. patent 3,681,421 to Barclay et al. there is disclosed the vapor phase catalytic ammoxidation of propylene over an oxide composition comprising antimony, vanadium and one or more additional polyvalent metals selected from tin, iron, cobalt and titanium in proportion 1 gm atom of antimony, 0.12 to 0.5 gm. atoms of vanadium and 0.25 to 0.5 gm atoms of each such additional polyvalent metal. This catalyst thus contains at least 2 atoms of Sb per atom of V, plus a minimum of 1/2 atom each of the foregoing polyvalent metals which is present, thus overlapping the empirical formulas of the compositions of the catalysts used in the method of the present invention. The empirical formula of Example 1 of Barclay et al. is VSb₂Sn, and of Example 3 is VSb₂Ti_{0.79} and thus overlap the empirical formula of the catalysts of the present invention. The catalysts used in the present method, however, are made in a different manner from the catalysts of the Barclay et al. patent that overlap the empirical formula of compositions of the catalysts of the present invention.

EP-A-31693 discloses using catalysts comprising a tin antimonate oxide complex of the formula:

AₐD_{b}Te_{d}SnₑSb_{f}Oₓ

- wherein: A represents one or more of the following elements: Cu, V, W and Mo;
D represents one or more of the following elements: Bi, Ti, Ge, Ce, La, Cr, Mn, Mg, Ca, Co, Ni, Nb, Ta, Ag, Zn, Cd, K, Cs, U, B, P and Eu;
and

- wherein: a is 0.001 to 10;
b is 0 to 10;
d is 0.001 to 10;
e is 0.1 to 10;
f is 1 to 20;
$\text{f > a + b + d + e}$; and
x is a number sufficient to satisfy the valence requirements of the other elements present.

Example 3 thereof describes adding V₂O₅ dissolved in water and a small amount of oxalic acid to a slurry prepared from Sb₂O₃, granular tin and Cu(NO₃)₂.3H₂O 85% WO₃ and (NH₄)₆ Mo₇ O₂₄ .4H₂O dissolved in water.

According to the present invention C₃ to C₅ mono-olefins are ammoxidized to a,b-mono-unsaturated acyclic nitriles having 3 to 5 carbon atoms by introducing such mono-olefins, molecular oxygen and ammonia into a reaction zone into vapor phase contact with a solid ammoxidation catalyst, wherein the mol ratio of introduced molecular oxygen and ammonia to said introduced mono-olefin is at least 1.5 and 1.0, respectively, wherein said catalyst contains the elements and proportions indicated by the empirical formula:

V₁SbₐMₘNₙOₓ

- where a =: 0.5 to 2
- M =: one or more of: Sn, Ti, Fe and Ga
- m =: 0.05 to 3, usually at least 0.1 and at most 1
- N =: one or more of: W, Bi, Mo, Li, Mg, P, Zn, Mn, Te, Ge, Nb, Zr, Cr, Al, Cu, Ce, B
- n =: 0.0 to 0.5, and
- x =: number of oxygen atoms required to satisfy the valence requirements of all the remaining elements,
and where the preparation of the catalyst includes firstly contacting in an aqueous dispersion only a vanadium compound and an antimony compound while said vanadium compound is in solution, heating the resulting slurry until the color of the slurry changes from yellow to green to black, adding components M and optionally N and calcining the catalyst at a temperature greater than 750°C.

The term "unsaturated" in the preceding paragraph means carbon-to-carbon olefinic unsaturation. The product nitriles contain no acetylenic unsaturation.

In the present process the C₃ to C₅ olefin fed to the reaction zone can contain up to 10 mole percent C₃ to C₅ paraffins, based on the total moles of olefins plus paraffins. In this way relatively inexpensive olefin feedstock streams can be used without being highly purified. Usually, however, the hydrocarbon feed to the reaction zone is almost entirely C₃ to C₅ olefins except for small amounts of impurities.

The importance of the last clause in the foregoing statement of the invention is illustrated in the examples. Ammoxidation of propylene with Example 28 catalyst, a repeat of Example 1 catalyst of Barclay et al. gave a productivity of 0.09 pounds of acrylonitrile per pound of catalyst per hour. This is to be compared with a productivity of 0.34 of a catalyst of the same empirical composition (Example 9) when used in ammoxidation of propylene, wherein the catalyst was prepared in the manner of the present invention. Or the comparisons can be made using the same catalyst composition, wherein a portion of the Example 9 catalyst which was not washed was used (Example 10). There the productivity was 0.30 pounds of acrylonitrile per pound of catalyst per hour.

The productivity of the actual Example 1 of Barclay et al. was calculated to be only 0.032 pounds of acrylonitrile per pound of catalyst per hour.

The catalysts of U.S. patent 5,008,427 to Brazdil et al. and the present catalysts greatly overlap. They are used for the ammoxidation of paraffins in a process calling for a large excess of propane over both O₂ and NH₃. It is disclosed also that minor portions of olefins (such as propylene) can be present in the feed, although no actual working examples do have olefins in the feed. The highest acrylonitrile productivity in the large number of specific examples was 0.068 pounds of acrylonitrile per pound of catalyst per hour.

In the preparation of the catalysts used in the present process the calcination temperature must be over 750oC, more usually at least 780oC. Usual calcination temperatures are in the range 790 to 1050oC. While not being limited to any particular theory, we believe that the M elements increase the activity and stability of the VSb0₄ crystalline phase which is formed in making the catalyst of the formula. While the high temperature calcination of the catalyst precursor is needed to bring about increased acrylonitrile selectivity of the present catalyst composition, the calcination temperatures over 750oC tend to cause decomposition of the active VSb0₄ phase and it is believed that the M elements not only increase the activity of the catalyst, but also perform the important function of stabilizing the active VSbO₄ crystalline phase.

While washing the finished, calcined catalyst with a lower alcohol, water, or an ammonium hydroxide solution usually somewhat increases the selectivity of conversion of the olefin, such as propylene to acrylonitrile, such treatments are not necessary.

The ammoxidation reaction is carried out at a temperature in the range from 350oC to 700oC, but is usually in the range 430 to 520oC. The latter range is especially useful in the ammoxidation of propylene to acrylonitrile, plus HCN.

In the ammoxidation process of the present invention, the reaction is carried out in the gaseous phase by contacting a mixture containing the olefin, ammonia and molecular oxygen, and inert diluent, if any, conveniently in a fixed bed of the catalyst or a gravity flowing bed, a fluidized bed or a fast transport reactor mode.

Examples of applicable inert gaseous diluents are N₂, He, CO₂ and H₂0. In the present process in all its embodiments the volume ratio of inert gaseous diluent to olefin fed to the reaction zone is usually in the range zero to 8 (more often zero to 4).

The average contact time can often be from 0.01 to 10 seconds, but is usually from 0.02 to 5 seconds, more usually from 0.1 to 2 seconds.

The pressure in the reaction zone usually ranges from just over atmospheric up to 75, more usually up to 50, psia.

Nothing in the prior art remotely suggested the desirability of using catalysts having the empirical composition of Barclay et al. made by a process of Brazdil et al., U.S. patent 5,008,427. We have made the truly astounding discovery, however, that the catalyst compositions coming within the empirical formula recited in the claims and made by the method of the claims can catalyze the ammoxidation of olefins such as propylene at 2 to 5 or more times the rate of productivity of the best commercial catalysts, which has a productivity rate of about 0.10 pound of acrylonitrile per pound of catalyst per hour. This result is achieved at yields of acrylonitrile of over 65 percent and propylene conversion rates well over 90 percent.

Such remarkable results were extremely surprising and certainly unpredictable in the absence of actual data. The results of the experiments were a completely unexpected discovery not even contemplated by us in advance.

Another advantage of the use of the present catalysts over commercial catalysts is that generally much less acrolein is produced, thus simplifying the process for recovery of product acrylonitrile and HCN.

The catalysts used in the propylene ammoxidation reactions summarized in the tables were made as noted in the following examples. Some of the catalysts after calcining were washed with water or with an alcohol, and then dried. In these specific examples, Nalco is The Nalco Chemical Company of Chicago, Illinois. Nyacol is Nyacol Products Inc., an affilate of the PQ Corporation of Ashland, MA. DeGussa is Degussa Corporation, Teterboro, N.J., a subsidiary of Degussa AG, Frankfurt, Germany. Nissan is Nissan Chemical Industries, Ltd. of Tokyo, Japan.

The washing step with an alcohol or with water, referred to in some of the specific examples was effected by placing the catalyst in a coarse glass frit funnel, pouring alcohol or water over the catalyst, stirring in order to spread the catalyst evenly over the bottom of the funnel, then allowing the stated amount of the alcohol or water to pass over the catalyst without suction. It will be noted that very large amounts of water were needed compared to the amount of an alcohol needed in the examples. When water was used, distilled water was used and washing was continued until the conductivity of the effluent wash water reached that of the distilled water.

### Example 1

16.33g V₂0₅ was added to a mixture consisting of 540cc H₂0 and 60.0g of 30% H₂0₂ in a 2-liter beaker, and stirred at RT about 15 minutes, until a dark red peroxy complex had formed. 31.24g Sb₂0₃ powder was then added, the hot plate temperature control was set to "high", and the beaker was covered with a watchglass. As it heated, the color of the slurry changed from yellow to green to black. The mixture was digested for approximately 3 hrs; water was added occasionally to keep the volume constant. 26.92g of a 20% Sn0₂ sol (Nalco 88SN123) was added, followed by 7.14g of fumed Ti0₂ (Degussa, P-25) and 100.0g of a 40% Si0₂ sol (Nalco 2327). The catalyst slurry was evaporated on the hot plate with constant stirring until it thickened. It was then dried at 120oC overnight and calcined at 290oC for 3 hours, at 425oC for 3 hours and at 650oC for 8 hours. After this, the catalyst was ground and screened to a 20-35 mesh size. The ground catalyst was given a final calcination at 810oC for 3 hours. Before being evaluated in the microreactor, 8.0g of the calcined catalyst was Soxhlet extracted with MeOH for 1.5 hours and then dried at 120oC. The catalyst composition was 60% VSb_{1.2}Sn_{0.2}Ti_{0.5}0ₓ - 40% Si0₂.

### Example 2

A catalyst of the composition VSb_{1.5}Oₓ was made as follows: 29.53g V₂0₅ was added to a mixture consisting of 900cc H₂0 and 100g of 30% H₂0₂ in a 2-liter beaker, and stirred at RT about 15 minutes, until a dark red peroxy complex had formed. 70.63g Sb₂0₃ powder was then added, the hot plate temperature control was set to "high", and the beaker was covered with a watchglass. As it heated, the color of the slurry changed from yellow to green to black. The mixture was digested for approximately 3 hours; water was added occasionally to keep the volume constant. The slurry was evaporated on the hot plate with constant stirring until it thickened. It was then dried at 120oC overnight and calcined at 650oC for 8 hours. After this, the catalyst was ground and screened to a 20-35 mesh size. The ground catalyst was given a final calcination at 810oC for 3 hours. Before being evaluated in the microreactor, 10g of the calcined catalyst was washed with 2 liters of water at RT and then dried at 120oC.

### Example 3

A catalyst of the composition VSb_{1.4}Sn_{0.2}Ti_{0.2}Oₓ was made as follows: 26.8g V₂0₅ was added to a mixture consisting of 900 cc H₂0 and 100g of 30% H₂0₂ in a 2-liter beaker, and stirred at RT about 15 minutes, until a dark red peroxy complex had formed. 59.82g Sb₂0₃ powder was then added, the hot plate temperature control was set to "high", and the beaker was covered with a watchglass. As it heated, the color of the slurry changed from yellow to green to black. The mixture was digested for approximately 3 hrs; water was added occasionally to keep the volume constant. 44.18g of a 20% Sn0₂ sol (Nalco 88SN123) was added, followed by 4.68g of Ti0₂ powder. The slurry was evaporated on the hot plate with constant stirring until it thickened. It was then dried at 120oC overnight.

The foregoing was repeated three times, and the four dried batches thoroughly blended into one large master batch. This was calcined at 650oC for 8 hours. After this, the catalyst was ground and screened to a 20-35 mesh size. The ground catalyst was given a final calcination at 790oC for 3 hours. Before being evaluated in a microreactor, 10.0g of the calcined catalyst was washed with 6 liters of water at RT and then dried at 120oC.

(Note that the washing of a portion of the catalyst was omitted. When tested in the ammoxidation of propylene, it proved to be an active catalyst, but less active than the washed catalyst of this example.)

### Example 4

A catalyst having the empirical composition VSb₂Sn_{0.5}Oₓ was made as follows: 7.93g V₂0₅ was added to a mixture consisting of 360cc H₂0 and 40g of 30% H₂0₂ in a 2-liter beaker, and stirred at RT about 15 minutes, until a dark red peroxy complex had formed. 25.30g Sb₂0₃ powder was then added, the hot plate temperature control was set to "high", and the beaker was covered with a watchglass. As it heated, the color of the slurry changed from yellow to green to black. The mixture was digested for approximately 3 hrs; water was added occasionally to keep the volume constant. 26.27g of a 24.9% Sn0₂ sol (Nyacol) was added. The slurry was evaporated on the hot plate with constant stirring until it thickened. It was then dried at 120oC overnight and calcined at 650oC for 8 hours. After this, the catalyst was ground and screened to a 20-35 mesh size. The ground catalyst was given a final calcination at 810oC for 3 hours. Before being evaluated in the microreactor, 8 g of the calcined catalyst was washed with 100cc isobutanol at RT and then dried at 120oC.

### Example 5

A catalyst having the empirical formula VSb_{1.4}Sn_{0.2}Ti_{0.1}Al_{0.001}Oₓ was made as follows: 27.43g V₂0₅ was added to a mixture consisting of 900 cc H₂0 and 100 g of 30% H₂0₂ in a 2-liter beaker, and stirred at RT about 15 minutes, until a dark red peroxy complex had formed. 61.25g Sb₂0₃ powder was then added, the hot plate temperature control was set to "high", and the beaker was covered with a watchglass. As it heated, the color of the slurry changed from yellow to green to black. The mixture was digested for approximately 3 hrs; water was added occasionally to keep the volume constant. 90.46 g of a 10% Sn0₂ sol (Nalco 1160) was added, followed by 2.4 g of Ti0₂ powder and 11.26g of a 1.0 wt % Al(NO₃)₃ · 9H₂0 solution. The slurry was evaporated on the hot plate with constant stirring until it thickened. It was then dried at 120oC overnight and calcined at 650oC for 8 hours. After this, the catalyst was ground and screened to a 20-35 mesh size. The ground catalyst was given a final calcination at 810oC for 3 hours. Before being evaluated in the microreactor, 8g of the calcined catalyst was washed with 75cc isobutanol at RT and then dried at 120oC.

### Example 6

A catalyst having the empirical formula 85% VSb_{1.1}Sn_{0.2} -15% Si0₂ was made as follows: 27.61g V₂0₅ was added to a mixture consisting of 900 cc H₂0 and 100g of 30% H₂0₂in a 2-liter beaker, and stirred at RT about 15 minutes, until a dark red peroxy complex had formed. 48.43g Sb₂0₃ powder was then added, the hot plate temperature control was set to "high", and the beaker was covered with a watchglass. As it heated, the color of the slurry changed from yellow to green to black. The mixture was digested for approximately 3 hrs; water was added occasionally to keep the volume constant. 85.08g of a 10.7% Sn0₂ sol (Nalco 1181D) was added, followed by 50 g of a 30% Si0₂ sol (Nalco). The slurry was evaporated on the hot plate with constant stirring until it thickened. It was then dried at 120oC overnight and calcined at 290oC for 3 hours, at 425oC for 3 hours and at 650oC for 8 hours. After this, the catalyst was ground and screened to a 20-35 mesh size. The ground catalyst was given a final calcination at 810oC for 3 hours. Before being evaluated in the microreactor, 8g of the calcined catalyst was Soxhlet extracted with methanol for 1.5 hours and then dried at 120oC.

### Example 7

A catalyst of the empirical formula VSb_{7.5}Cr_{2.5}Oₓ was made as follows: 3.33g V₂0₅ was added to a mixture consisting of 60cc H₂0 and 30g of 30% H₂0₂ in a 2-liter beaker, and stirred at RT about 15 minutes, until a dark red peroxy complex had formed. Then 450cc of H₂0 was added, followed by 39.78g Sb₂0₃ powder, after which heating was continued for 30 minutes. Then 9.1g Cr0₃ was added and heating continued for another 2 hours. This slurry was evaporated on the hot plate with constant stirring until it thickened. It was then dried at 120oC overnight and calcined at 650oC for 8 hours. After this, the catalyst was ground and screened to a 20-35 mesh size. The ground catalyst was given a final calcination at 810oC for 3 hours.

### Example 8

A catalyst having the empirical composition VSb₁₂Ti₁₅Oₓ was made as follows: 1.5g V₂0₅ was added to a mixture consisting of 30 cc H₂0 and 15g of 30% H₂0₂ in a 2-liter beaker, and stirred at RT about 15 minutes, until a dark red peroxy complex had formed. Then 450 cc of H₂0 and 28.78g of Sb₂0₃ powder were added and the slurry heated for 30 minutes. Then 19.72g of Ti0₂ powder slurried in a small amount of water was added and heating continued for 2 more hours. The slurry was evaporated on the hot plate with constant stirring until it thickened. It was then dried at 120oC overnight and calcined at 650oC for 8 hours. After this, the catalyst was ground and screened to a 20-35 mesh size. The ground catalyst was given a final calcination at 810oC for 3 hours.

### Example 9

A catalyst of the empirical composition VSb₂Sn0ₓ was made as follows: 17.14g V₂0₅ was added to a mixture consisting of 900 cc H₂0 and 100g of 30% H₂0₂ in a 2-liter beaker, and stirred at RT about 15 minutes, until a dark red peroxy complex had formed. 54.68g Sb₂0₃ powder was then added, the hot plate temperature control was set to "high", and the beaker was covered with a watchglass. As it heated, the color of the slurry changed from yellow to green to black. The mixture was digested for approximately 3 hrs; water was added occasionally to keep the volume constant. 264.17g of a 10.7% Sn0₂ sol (Nalco 1181D) was added. The slurry was evaporated on the hot plate with constant stirring until it thickened. It was then dried at 120oC overnight and calcined at 650oC for 8 hours. After this, the catalyst was ground and screened to a 20-35 mesh size. The ground catalyst was given a final calcination at 810oC for 3 hours. Before being evaluated in the microreactor, 8g of the calcined catalyst washed with 100cc isobutanol at RT and then dried at 120oC.

### Example 10

This catalyst is a portion of the catalyst of the last example that was not washed.

### Example 11

A catalyst having the empirical composition VSb_{1.2}Sn_{0.2}Ti_{0.5}Oₓ was made as follows: 27.21g V₂0₅ was added to a mixture consisting of 900 cc H₂0 and 100g of 30% H₂0₂ in a 2-liter beaker, and stirred at RT about 15 minutes, until a dark red peroxy complex had formed. 52.07g Sb₂0₃ powder was then added, the hot plate temperature control was set to "high", and the beaker was covered with a watchglass. As it heated, the color of the slurry changed from yellow to green to black. The mixture was digested for approximately 3 hrs; water was added occasionally to keep the volume constant. 83.85g of a 10.7% Sn0₂ sol (Nalco 1181D) was added, followed by 11.89g of fumed Ti0₂ (Degussa, P-25). The slurry was evaporated on the hot plate with constant stirring until it thickened. It was then dried at 120oC overnight and calcined at 650oC for 8 hours. After this, the catalyst was ground and screened to a 20-35 mesh size. The ground catalyst was given a final calcination at 810oC for 3 hours. Before being evaluated in the microreactor, 8g of the calcined catalyst was washed with 75cc isobutanol at RT and then dried at 120oC.

### Example 12

This catalyst is a portion of the catalyst of the previous example that was not washed.

### Example 13

A catalyst having the empirical formula VSb_{1.2}Sn_{0.2} TiOₓ was made as follows: 24.32g V₂0₅ was added to a mixture consisting of 900cc H₂0 and 100 g of 30% H₂0₂ in a 2-liter beaker, and stirred at RT about 15 minutes, until a dark red peroxy complex had formed. 46.53g Sb₂0₃ powder was then added, the hot plate temperature control was set to "high", and the beaker was covered with a watchglass. As it heated, the color of the slurry changed from yellow to green to black. The mixture was digested for approximately 3 hrs; water was added occasionally to keep the volume constant. 74.94 g of a 10.7% Sn0₂ sol (Nalco 1181D) was added, followed by 21.26g of fumed Ti0₂ (Degussa, P-25). The catalyst slurry was evaporated on the hot plate with constant stirring until it thickened. It was then dried at 120oC overnight and calcined at 650oC for 8 hours. After this, the catalyst was ground and screened to a 20-35 mesh size. The ground catalyst was given a final calcination at 810oC for 3 hours. Before being evaluated in the microreactor, 8.0g of the calcined catalyst was washed with 150cc isobutanol at RT and then dried at 120oC.

### Example 14

A catalyst having the empirical formula 85% VSb_{1.2}Sn_{0.2}Ti_{0.5}Oₓ - 15%Si0₂ was made as follows: 23.13g V₂0₅ was added to a mixture consisting of 900cc H₂0 and 100g of 30% H₂0₂ in a 2-liter beaker, and stirred at RT about 15 minutes until a dark red peroxy complex had formed. 44.26g Sb₂0₃ powder was then added, the hot plate temperature control was set to "high", and the beaker was covered with a watchglass. As it heated, the color of the slurry changed from yellow to green to black. The mixture was digested for approximately 3 hrs; water was added occasionally to keep the volume constant. 71.27g of a 10.7% Sn0₂ sol (Nalco 1181D) was added followed by 10.11g of fumed Ti0₂ (Degussa, P-25) and 50.0g of a 30% Si0₂ sol (Nalco). The catalyst slurry was evaporated on the hot plate with constant stirring until it thickened. It was then dried at 120oC overnight and calcined at 650oC for 8 hours. After this, the catalyst was ground and screened to a 20-35 mesh size. The ground catalyst was given a final calcination at 810oC for 3 hours. Before being evaluated in the microreactor, 8g of the calcined catalyst was washed by Soxhlet extraction with methanol and then dried at 120oC.

### Example 15

A catalyst having the empirical formula 60% VSb_{1.2}Sn_{0.2}Ti_{0.5}Oₓ - 40% Si0₂ was made as follows: 16.33 g V₂0₅ was added to a mixture consisting of 540cc H₂0 and 60g of 30% H₂0₂ in a 2-liter beaker, and stirred at RT about 15 minutes, until a dark red peroxy complex had formed. 31.24g Sb₂0₃ powder was then added, the hot plate temperature control was set to "high", and the beaker was covered with a watchglass. As it heated, the color of the slurry changed from yellow to green to black. The mixture was digested for approximately 3 hrs; water was added occasionally to keep the volume constant. 50.31g of a 10.7% Sn0₂ sol (Nalco 1181D) was added, followed by 7.14 g of fumed Ti0₂ (Degussa, P-25) and 133.3 g of a 30% Si0₂ sol (Nalco). The catalyst slurry was evaporated on the hot plate with constant stirring until it thickened. It was then dried at 120oC overnight and calcined at 650oC for 8 hours. After this, the catalyst was ground and screened to a 20-35 mesh size. The ground catalyst was given a final calcination at 810oC for 3 hours. Before being evaluated in the microreactor, 8.0g of the calcined catalyst was washed with 150cc isobutanol at RT and then dried at 120oC.

### Example 16

A catalyst having the empirical formula VSb_{1.2}Sn_{0.2}Ti_{0.5}Oₓ was made as follows: 17.41g NH₄V0₃ were dissolved in 1 liter of hot water. 26.03g Sb₂0₃ powder was added and the slurry was refluxed for about 3 hours. 41.92g of a 10.7% Sn0₂ sol (Nalco 1181D) and 5.95 g of fumed Ti0₂ (Degussa, P-25) were then added. The slurry was evaporated on a hot plate with constant stirring until it thickened. It was then dried at 120oC overnight and calcined at 650oC for 8 hours. After this, the catalyst was ground and screened to a 20-35 mesh size. The ground catalyst was given a final calcination at 810oC for 3 hours. Before being evaluated in the microreactor, 8.0g of the calcined catalyst was washed with 100cc isobutanol at RT and then dried at 120oC.

### Example 17

A portion of the calcined catalyst of the last example was Soxhlet extracted with methanol for 1.5 hours instead of being washed with isobutanol.

### Example 18

A catalyst having the empirical formula 60% VSb_{1.2}Sn_{0.2}Ti_{0.75}Oₓ - 40% Si0₂ was made as follows: 15.41g V₂0₅ was added to a mixture consisting of 540 cc H₂0 and 60g of 30% H₂0₂ in a 2-liter beaker, and stirred at RT about 15 minutes, until a dark red peroxy complex had formed. 29.49g Sb₂0₃ powder was then added, the hot plate temperature control was set to "high", and the beaker was covered with a watchglass. As it heated, the color of the slurry changed from yellow to green to black. The mixture was digested for approximately 3 hrs; water was added occasionally to keep the volume constant. 25.40g of a 20% Sn0₂ sol (Nalco 88SN123) was added, followed by 10.1g of fumed Ti0₂ (Degussa, P-25) and 133.33g of a 30% Si0₂ sol (Nalco). The slurry was evaporated on the hot plate with constant stirring until it thickened. It was then dried at 120oC overnight and calcined at 290oC for 3 hours, at 425oC for 3 hours and at 650oC for 8 hours. After this, the catalyst was ground and screened to a 20-35 mesh size. The ground catalyst was given a final calcination at 810oC for 3 hours. Before being evaluated in the microreactor, 8g of the calcined catalyst was washed with 125cc isobutanol at RT and then dried at 120oC.

### Example 19

A catalyst having the empirical formula VSb_{1.2} Sn_{0.2}Ti_{0.5}Oₓ was made as follows: a solution of 17.41g NH₄V0₃ dissolved in 600cc hot water was added dropwise over a period of 2 hours to 26.03g Sb₂0₃ powder slurried in 400cc water. The mixture was digested for 1 hour. Then 37.38g of a 12% Sn0₂ sol (Nalco 1188) and 5.95g of fumed Ti0₂ (Degussa, P-25) were added. The slurry was evaporated on a hot plate with constant stirring until it thickened. It was then dried at 120oC overnight and calcined at 650oC for 8 hours. After this, the catalyst was ground and screened to a 20-35 mesh size. The ground catalyst was given a final calcination at 810oC for 3 hours. Before being evaluated in the microreactor, 8.0 g of the calcined catalyst was washed by Soxhlet extraction with methanol for 1.5 hours and then dried at 120oC.

### Example 20

A catalyst having the empirical formula 60% VSb_{1.2}Sn_{0.2}Ti_{0.5}K_{0.001}Oₓ - 40% Si0₂ was made as follows: 16.3g V₂0₅ was added to a mixture consisting of 540cc H₂0 and 60.0g of 30% H₂0₂ in a 2-liter beaker, and stirred at RT about 15 minutes, until a dark red peroxy complex had formed. 31.24g Sb₂0₃ powder was then added, the hot plate temperature control was set to "high", and the beaker was covered with a watchglass. As it heated, the color of the slurry changed from yellow to green to black. The mixture was digested for approximately 3 hrs; water was added occasionally to keep the volume constant. A drop of the slurry was then tested on a filter paper to see whether it would bleed; since it did not, 26.9g of a 20% Sn0₂ sol (Nalco 88SN123) was added, followed by 7.13g of fumed Ti0₂ (Degussa, P-25) 0.18g 10% KN0₃ solution in water and 100.0g of a 40% Si0₂ sol (Nalco 2327). The slurry was evaporated on the hot plate with constant stirring until it thickened. It was then dried at 120oC overnight and calcined at 290oC for 3 hours, at 425oC for 3 hours and at 650oC for 8 hours. After this, the catalyst was ground and screened to a 20-35 mesh size. The ground catalyst was given a final calcination at 810oC for 3 hours. Before being evaluated in the microreactor, 8.0g of the calcined catalyst was washed with 100cc isobutanol at RT and then dried at 120oC.

### Example 21

A catalyst having the empirical formula 60% VSb_{1.2}Sn_{0.2}Ti_{0.5} - 40% Si0₂ was made as follows: 27.21g V₂0₅ was added to a mixture consisting of 900 cc H₂0 and 100g of 30% H₂0₂ in a 2-liter beaker, and stirred at RT about 15 minutes, until a dark red peroxy complex had formed. 52.07g Sb₂0₃ powder was then added, the hot plate temperature control was set to "high", and the beaker was covered with a watchglass. As it heated, the color of the slurry changed from yellow to green to black. The mixture was digested for approximately 3 hrs; water was added to keep the volume constant. 83.85g of a 10.7% Sn0₂ Sol (Nalco 1181D) was added, followed by 11.89g of fumed Ti0₂ (Degussa, P-25). The slurry was digested for 0.5 hr. and then there was added 222 g of a 30% Si0₂ sol (Nissan). The slurry was evaporated on the hot plate with constant stirring until it thickened. It was then dried at 120oC overnight and calcined at 290oC for 3 hours, at 425oC for 3 hours and at 650oC for 8 hours. After this, the catalyst was ground and screened to a 20-35 mesh size. The ground catalyst was given a final calcination at 810oC for 3 hours. Before being evaluated in the microreactor, 8g of the calcined catalyst was washed with 100cc isobutanol at RT and then dried at 120oC.

### Example 22

A catalyst having the empirical formula 60% VSb_{1.2}Sn_{0.2}Ti_{0.5}Oₓ - 40% Si0₂ was made as follows: 16.33g V₂0₅ was added to a mixture consisting of 540cc H₂0 and 60.0g of 30% H₂0₂ in a 2-liter beaker, and stirred at RT about 15 minutes, until a dark red peroxy complex had formed. 31.24g Sb₂0₃ powder was then added, the hot plate temperature control was set to "high", and the beaker was covered with a watchglass. As it heated, the color of the slurry changed from yellow to green to black. The mixture was digested for approximately 3 hrs; water was added occasionally to keep the volume constant. 26.92g of a 20% Sn0₂ sol (Nalco 88SN123) was added, followed by 7.14g of powdered Ti0₂ and 133.33g of a 30% Si0₂ sol (Nissan). The catalyst slurry was evaporated on the hot plate with constant stirring until it thickened. It was then dried at 120oC overnight and calcined at 290oC for 3 hours, at 425oC for 3 hours and at 650oC for 8 hours. After this, the catalyst was ground and screened to a 20-35 mesh size. The ground catalyst was given a final calcination at 810oC for 3 hours. Before being evaluated in the microreactor, 8.0g of the calcined catalyst was washed with 100cc isobutanol at RT and then dried at 120oC.

### Example 23

A catalyst having the empirical formula 60% VSb_{1.2}Sn_{0.2}Ti_{0.5} - 40% Si0₂ was made as follows: 16.33g V₂0₅ was added to a mixture consisting of 540cc H₂0 and 60.0g of 30% H₂0₂ in a 2-liter beaker, and stirred at RT about 15 minutes, until a dark red peroxy complex had formed. 31.24g Sb₂0₃ powder was then added, the hot plate temperature control was set to "high", and the beaker was covered with a watchglass. As it heated, the color of the slurry changed from yellow to green to black. The mixture was digested for approximately 3 hrs; water was added occasionally to keep the volume constant. 26.92g of a 20% Sn0₂ sol (Nalco 88SN123) was added, followed by 7.14g of fumed Ti0₂ (Degussa, P-25) and 117.65g of a 34% Si0₂ sol (Nalco H-1034). The catalyst slurry was evaporated on the hot plate with constant stirring until it thickened. It was then dried at 120oC overnight and calcined at 290oC for 3 hours, at 425oC for 3 hours and at 650oC for 8 hours. After this, the catalyst was ground and screened to a 20-35 mesh size. The ground catalyst was given a final calcination at 810oC for 3 hours. Before being evaluated in the microreactor, 8.0g of the calcined catalyst was washed with 100cc isobutanol at RT and then dried at 120oC.

### Example 24

A catalyst having the empirical formula 60% VSbSn_{0.2}Ti_{0.5}Oₓ - 40% Si0₂ was made as follows: 17.88g V₂0₅ was added to a mixture consisting of 540cc H₂0 and 60g of 30% H₂0₂ in a 2-liter beaker, and stirred at RT about 15 minutes, until a dark red peroxy complex had formed. 28.51g Sb₂0₃ powder was then added, the hot plate temperature control was set to "high", and the beaker was covered with a watchglass. As it heated, the color of the slurry changed from yellow to green to black. The mixture was digested for approximately 3 hrs; water was added occasionally to keep the volume constant. 29.47g of a 20% Sn0₂ sol (Nalco 88SN123) was added, followed by 7.81g of fumed Ti0₂ (Degussa, P-25) and 133.33g of a 30% Si0₂ sol (Nissan). The catalyst slurry was evaporated on the hot plate with constant stirring until it thickened. It was then dried at 120oC overnight and calcined at 290oC for 3 hours, at 425oC for 3 hours and at 650oC for 8 hours. After this, the catalyst was ground and screened to a 20-35 mesh size. The ground catalyst was given a final calcination at 810oC for 3 hours. Before being evaluated in the microreactor, 8g of the calcined catalyst was washed with 100cc isobutanol at RT and then dried at 120oC.

### Example 25

This catalyst was prepared similarly to the first example except on a large scale. It had the same composition as that example. However, the procedure differed after formation of the slurry, which was concentrated somewhat by evaporation and then was spray dried in a pilot plant size spray dryer to make fluidizable microspheroidal catalyst. 4.5 pounds of the catalyst was recovered from the chamber and 3.5 pounds from the cyclone.

A 500 gram sample of the chamber product was calcined at 290oC for 3 hours, 425oC for 3 hours, 650oC for 8 hours and 810oC for 3 hours. 100 grams of this catalyst was washed with 1500 cc of isobutanol, and thereafter dried.

### Example 26

A catalyst having the empirical formula 60% VSb_{1.2}Sn_{0.2}Ti_{0.5}Oₓ was made as follows: 16.33g V₂0₅ was added to a mixture consisting of 900cc H₂0 and 100g of 30% H₂0₂ in a 2-liter beaker, and stirred at RT about 15 minutes, until a dark red peroxy complex had formed. 31.24g Sb₂0₃ powder was then added, the hot plate temperature control was set to "high" and the beaker was covered a watchglass. As it heated, the color of the slurry changed from yellow to green to black. The mixture was digested for approximately 3 hrs; water was added occasionally to keep the volume constant. 50.3g of a 20% Sn0₂ sol (Nalco 88SN123) was added, followed by 7.14g of fumed Ti0₂ (Degussa, P-25) and 133.3 g of a 30% Si0₂ sol (Nalco). The catalyst slurry was evaporated on the hot plate with constant stirring until it thickened. It was then dried at 120oC overnight and calcined at 290oC for 3 hours, at 425oC for 3 hours and at 650oC for 8 hours. After this, the catalyst was ground and screened in a 20-35 mesh size. The ground catalyst was given a final calcination at 810oC for 3 hours. Before being evaluated in the microreactor, 8.0g of the calcined catalyst was washed with 100cc isobutanol at RT and then dried at 120oC.

### Example 27

A catalyst having the empirical formula 80% VSb_{1.2}Ga_{0.2}Ti_{0.5}Oₓ - 20% SiO₂ was made as follows: 5.63 V₂0₅ was added to a mixture consisting of 450cc H₂0 and 50.0g of 30% H₂0₂ in a beaker, and stirred at RT about 15 minutes, until a dark red peroxy complex had formed. 10.78g Sb₂0₃ powder was then added, the hot plate temperature control was set to "high", and the beaker was covered with a watchglass. As it heated, the color of the slurry changed form yellow to green to black. The mixture was digested for approximately 3 hrs; water was added occasionally to keep the volume constant. After dissolving in a small amount of water, 5.15g of Ga(NO₃)₃·₉H₂0 was added, followed by 2.46g of fumed Ti0₂ (Degussa, P-25) and 16.67g of a 30% Si0₂ sol (Nissan). The catalyst slurry was evaporated on the hot plate with constant stirring until it thickened. It was then dried at 120oC over the weekend and calcined at 290oC for 3 hours, at 425oC for 3 hours and at 650oC for 8 hours. After this, the catalyst was ground and screened to a 20-35 mesh size. The ground catalyst was given a final calcination at 810oC for 3 hours. Before being evaluated in the microreactor, 8.0g of the calcined catalyst was washed with 75cc isobutanol at room temperature and then dried at 120oC.

### Example 28

A catalyst having the empirical formula VSb₂Sn0ₓ was prepared as follows: 46.64g of Sb₂0₃ powder was added during a period of 10 minutes to a stirred mixture of 240g H₂0 and 80g concentrated (70%) HN0₃, at 95-100oC. 18.99g of Sn metal powder (325 mesh size) was then added to the stirred suspension over a period of 30 minutes, while the temperature was maintained at 95-100oC. Stirring at this temperature was continued for another 15 minutes; the slurry was then cooled and centrifuged. The solid cake was resuspended in 240g H₂0 at 40oC. The pH of the slurry was raised to 7.0 by the addition of ^{∼}80cc of a NH₄OH solution consisting of 1/3 of 28% NH₄OH and 2/3 H₂0. The slurry was stirred for another 5 minutes, the pH was readjusted to 7.0 and the slurry was centrifuged again. The solid cake was re-suspended in 240cc H₂0 and recentrifuged. 14.63g of V₂0₅ powder was added to the solid cake and mixed thoroughly. The wet paste was dried in a thin layer at 120oC overnight. The dry powder was calcined at 290oC - 2 hrs., 425oC - 2 hrs., 650oC - 3 hrs. and at 850oC - 16 hrs.

### Example 29

This is a portion of the catalyst of the preceding example that was washed with isobutanol, using 75cc to wash 8g of catalyst. It was again dried at 120oC overnight.

### Example 30

A catalyst having the empirical formula 60% VSb_{1.2}Sn_{0.2}Ti_{0.5}Oₓ - 40% Si0₂ was made as follows: 8.16g V₂0₅ was added to a mixture consisting of 450cc H₂0 and 50g of 30% H₂0₂ in a 2-liter beaker, and stirred at RT about 15 minutes, until a dark red peroxy complex had formed. 15.62g Sb₂0₃ powder was then added, the hot plate temperature control was set to "high", and the beaker was covered with a watchglass. As it heated, the color of the slurry changed from yellow to green to black. The mixture was digested for approximately 3 hrs; water was added occasionally to keep the volume constant. 13.46g of a 20% Sn0₂ sol (Nalco 88SN123) was added, followed by 3.57g of fumed Ti0₂ (Degussa, P-25) and 33.33g of a 30% Si0₂ sol (Nissan) and 10.0g of fumed (Aerosil) Si0₂. The catalyst slurry was evaporated on the hot plate with constant stirring until it thickened. It was then dried at 120oC overnight and calcined at 290oC for 3 hours, at 425oC for 3 hours and at 650oC for 8 hours. After this, the catalyst was ground and screened to a 20-35 mesh size. The ground catalyst was given a final calcination at 810oC for 3 hours. Before being evaluated in the microreactor, 8g of the calcined catalyst was washed with 100cc isobutanol at RT and then dried at 120oC.

### Example 31

A catalyst having the empirical formula VSb_{1.2}SnTiOₓ was made as follows: 9.21g V₂0₅ was added to a mixture consisting of 450cc H₂0 and 50g of 30% H₂0₂ in a 2-liter beaker, and stirred at RT about 15 minutes, until a dark red peroxy complex had formed. 17.62g Sb₂0₃ powder was then added, the hot plate temperature control was set to "high", and the beaker was covered with a watchglass. As it heated, the color of the slurry changed from yellow to green to black. The mixture was digested for approximately 3 hrs; water was added occasionally to keep the volume constant. 75.89g of a 20% Sn0₂ sol (Nalco 88SN123) was added, followed by 8.05g of fumed Ti0₂ (Degussa, P-25). The catalyst slurry was evaporated on the hot plate with constant stirring until it thickened. It was then dried at 120oC overnight and calcined at 290oC for 3 hours, at 425oC for 3 hours and at 650oC for 8 hours. After this, the catalyst was ground and screened to a 20-35 mesh size. The ground catalyst was given a final calcination at 810oC for 3 hours. Before being evaluated in the microreactor, 8g of the calcined catalyst was washed with 100cc isobutanol at RT and then dried at 120oC.

### Example 32

A catalyst having the empirical composition VSb_{1.2}Sn_{0.2}Ti_{0.5}Oₓ was made as follows: 27.21g V₂0₅ was added to a mixture consisting of 900 cc H₂0 and 100g of 30% H₂0₂ in a 2-liter beaker, and stirred at RT about 15 minutes, until a dark red peroxy complex had formed. 52.07g Sb₂0₃ powder was then added, the hot plate temperature control was set to "high", and the beaker was covered with a watchglass. As it heated, the color of the slurry changed from yellow to green to black. The mixture was digested for approximately 3 hrs; water was added occasionally to keep the volume constant. 83.85g of a 10.7% Sn0₂ sol (Nalco 1181D) was added, followed by 11.89g of fumed Ti0₂ (Degussa, P-25). The slurry was evaporated on the hot plate with constant stirring until it thickened. It was then dried at 120oC overnight and calcined at 650oC for 8 hours. After this, the catalyst was ground and screened to a 20-35 mesh size. The ground catalyst was given a final calcination at 810oC for 3 hours. Before being evaluated in the microreactor, 8g of the calcined catalyst was washed with 75cc isobutanol at RT and then dried at 120oC.

### Example 33

A catalyst having the empirical formula VSb_{1.2}Sn_{0.5}Ti_{0.5} was made as follows: 11.99g V₂0₅ was added to a mixture consisting of 450cc H₂0 and 50g of 30% H₂0₂ in a 2-liter beaker, and stirred at RT about 15 minutes, until a dark red peroxy complex had formed. 22.95g Sb₂0₃ powder was then added, the hot plate temperature control was set to "high", and the beaker was covered with a watchglass. As it heated, the color of the slurry changed form yellow to green to black. The mixture was digested for approximately 3 hrs; water was added occasionally to keep the volume constant. 49.42g of a 20% Sn0₂ sol (Nalco 88SN123) was added, followed by 5.24g of fumed Ti0₂ (Degussa, P-25). The catalyst slurry was evaporated on the hot plate with constant stirring until, it thickened. It was then dried at 120oC overnight and calcined at 290oC for 3 hours, at 425oC for 3 hours and at 650oC for 8 hours. After this, the catalyst was ground and screened to a 20-35 mesh size. The ground catalyst was given a final calcination at 810oC for 3 hours. Before being evaluated in the microreactor, 8g of the calcined catalyst was washed with 100cc isobutanol at RT and then dried at 120oC.

### Example 34

A catalyst having an empirical composition 70% VSbSn_{0.2}Oₓ was made as follows: 13.46g of V₂0₅ was added to a mixture consisting of 450ml water and 50g of 30% H₂0₂ in a 1 liter beaker, and stirred at RT about 15 minutes, until a dark red peroxy complex had formed. 32.18g of Sb₂0₃ powder were then added, the hot plate temperature control was set to "high", and the beaker was covered with a watch glass. As it heated, the color of the slurry changed from yellow to green to black. the mixture was digested for approximately 3 hours; water was added occasionally to keep the volume constant. 17.82g of 25% Sn0₂ sol (Nyacol) were added and the slurry heated an additional 1 hour. The slurry was then cooled and centrifuged free of unreacted Sb₂0₃ to give a black aqueous sol with 10.02% solids content.

123.1g of the above were combined with 9.23g of 40% Si0₂ sol (Nalco 41D01) and evaporated on a hot plate and then dried over night at 120oC to give 15.58g. This solid was further heated at 425oC for 3 hours and 650oC for 8 hours. The solid was then ground to give 7.61g -20+35 mesh particles. This solid was finally heated at 810oC for 3 hours and then washed with 22 liters of water.

### Example 35

A catalyst having an empirical composition 60% VSb_{1.4}Sn_{0.2}T_{10.5} - 40% was made as follows: 15.02g of V₂0₅ was added to a mixture consisting of 540cc H₂0 and Si0₂ and 60g of 30% H₂0₂ in a 2-liter beaker, and stirred at RT about 15 minutes, until a dark red peroxy complex had formed. 33.54g Sb₂0₃ powder was then added, the hot plate temperature control was set to "high", and the beaker was covered with a watchglass. At it heated, the color of the slurry changed from yellow to green to black. The mixture was digested for approximately 3 hrs; water was added occasionally to keep the volume constant. 24.77g of a 20% Sn0₂ sol (Nalco 88SN123) was added, followed by 6.57g of fumed Ti0₂ (Degussa, P-25) and 133.33g of a 30% Si0₂ sol (Nissan). The catalyst slurry was evaporated on the hot plate with constant stirring until it thickened. It was then dried at 120oC overnight and calcined at 290oC for 3 hours, at 425oC for 3 hours and at 650oC for 8 hours. After this, the catalyst was ground and screened to a 20-35 mesh size. The ground catalyst was given a final calcination at 810oC for 3 hours. Before being evaluated in the microreactor, 8g of the calcined catalyst was washed with 100cc isobutanol at RT and then dried at 120oC.

### Example 36

A catalyst having the empirical formula 85% VSb_{1.2}Sn_{0.5}Ti_{0.5}Oₓ was made as follows: 10.19 g V₂0₅ was added to a mixture consisting of 450cc H₂0 and 50.0 g of 30% H₂0₂ in a beaker and stirred at RT about 15 minutes, until a dark red peroxy complex had formed. 19.50 g Sb₂0₃ powder was then added, the hot plate temperature control was set to "high", and the beaker was covered with a watchglass. As it heated, the color of the slurry changed from yellow to green to black. The mixture was digested for approximately 3 hrs; water was added occasionally to keep the volume constant. 42.01 g of a 20% Sn0₂ sol (Nalco 88SN123) was added, followed by 4.45g of fumed Ti0₂ (Degussa, P-25) and 25.0g of a 30% Si0₂ sol (Nissan). The catalyst slurry was evaporated on the hot plate with constant stirring until it thickened. It was then dried at 120oC overnight and calcined at 290oC for 3 hours, at 425oC for 3 hours and at 650oC for 8 hours. After this, the catalyst was ground and screened to a 20-35 mesh size. The ground catalyst was given a final calcination at 810oC for 3 hours. Before being evaluated in the microreactor, 8.0g of the calcined catalyst was washed with 125cc of isobutanol and then dried at 120oC.

### Example 37

A catalyst of the empirical composition 80% VSb_{1.2}Fe_{0.2}Ti_{0.5} - 20% Si0₂ was made as follows: 11.42g V₂0₅ was added to a mixture consisting of 450cc H₂0 and 50g of 30% H₂0₂ in a 2-liter beaker, and stirred at RT about 15 minutes, until a dark red peroxy complex had formed. 21.85 Sb₂0₃ powder was then added, the hot plate temperature control was set to "high", and the beaker was covered with a watchglass. As it heated, the color of the slurry changed from yellow to green to black. The mixture was digested for approximately 3 hours; water was added occasionally to keep the volume constant. Thereafter, 4.35g ferrous acetate, 4.99g of fumed Ti0₂ (Degussa, P-25) and 33.33g of a 30% Si0₂ sol (Nissan) were added. The catalyst slurry was evaporated on the hot plate with constant stirring until it thickened. It was then dried at 120oC overnight and calcined at 290oC for 3 hours, at 425oC for 3 hours and at 650oC for 8 hours. After this, the catalyst was ground and screened to a 20-35 mesh size. The ground catalyst was given a final calcination at 810oC for 3 hours. Before being evaluated in the microreactor, 8.0g of the calcined catalyst was washed with 100cc of isobutanol and then dried at 120oC.

### Example 38

A catalyst of the empirical formula 80% VSb_{1.2}Cr_{0.2}Ti_{0.5}Oₓ was made in the manner of the preceding example except that Cr0₃ was substituted for the ferrous acetate in an amount to satisfy the empirical formula.

### Example 39

A catalyst having the empirical composition VSb_{1.4}Sn_{0.2}Ti_{0.3}Oₓ was made as follows: 26.10g V₂0₅ was added to a mixture consisting of 400cc H₂0 and 100g of 30% H₂0₂ in a 2-liter beaker, and stirred at RT about 15 minutes, until a dark red peroxy complex had formed. 58.45g Sb₂0₃ powder was then added, the hot plate temperature control was set to "high", and the beaker was covered with a watchglass. As it heated, the color of the slurry changed from yellow to green to black. The mixture was digested for approximately 3 hrs; water was added occasionally to keep the volume constant. 42.12g of a 20.58%d Sn0₂ sol was added, followed by 6.87g of fumed Ti0₂ (Degussa, P-25). The catalyst slurry was evaporated on the hot plate with constant stirring until it thickened. It was then dried at 120oC overnight and calcined at 650oC for 8 hours. After this, the catalyst was ground and screened to a 20-35 mesh size. The ground catalyst was given a final calcination at 810oC for 3 hours. Before being evaluated in the microreactor, 8.0 g of the calcined catalyst was washed with 100cc of isobutanol and then dried at 120oC.

The Sn0₂ sol used in the above preparation of this catalyst was made as follows: 1800ml water and 900ml concentrated nitric acid were placed in a 4 liter beaker fitted with a magnetic stirrer and thermometer. 300g Sn metal (-20 mesh) were added over 2-2.5 hours maintaining the reaction temperature at 55-60C (not to exceed 60C). The white suspensions were stirred at room temperature overnight and then centrifuged. This separated the Sn0₂ well and allowed the supernatant liquid to be poured off without disturbing the solids. The combined solids were suspended in water (^{∼}2.5 liters total volume). Then, the suspension was brought to pH 7 by adding 380ml concentrated NH40H. The suspension was diluted to 3500ml and stirred overnight. The suspension was then centrifuged again and the solids were removed, combined and diluted to 3500ml with fresh water (first water wash). This suspension was stirred overnight and then centrifuged a third time. The water was discarded and the solids washed a second time with water (second wash) by resuspension in the centrifuge bottles. After shaking to disperse the solid, the bottles were matched in weight and centriguted a fourth time. The clear water wash was discarded. The wash water still indicated >0.5g/liter nitrate ion using JPB's test paper from Aldrich. Consequently, a third wash was done by resuspending the solid in water in the centrifuge bottles. This wash water was a dilute milky sol after centrifuging. Solids were only 0.113% and the test paper indicated approximately 1g nitrate/liter (diluted the wash 1:10 before using the paper). Further washing would peptize more Sn0₂ so the process was stopped at this point. Recovered 1564.2g of wet cake Sn0₂ after the final wash. Made up a 20% sol by adding 117.8g 40% methylamine solution to the wet solid, triturating to obtain a translucent grey paste and diluting the paste with 2122g water. The trituration was not easy because of the formation of chunks of solid partly penetrated by the amine solution. These chunks were sticky, slippery and difficult to disperse. The more difficult chunks were removed from the beaker and macerated in a large evaporating dish using the bottom of a beaker to smash the lumps. The sol had some grey unreacted Sn metal in suspension and was given a final 30 minute centrifuging. This separated the metal, but the sol could not be decanted away cleanly. Consequently, the sol was filtered through glass microfiber filter paper on a buchner funnel to remove this suspended metal. Final sol was light grey-green with solids 20.58%.

### Example 40

A pre-catalyst slurry of the composition of Example 1 was prepared as in that example except on a large scale. Then a small portion of this catalyst slurry was evaporated on the hot plate with constant stirring until it thickened. It was then dried at 120oC overnight and calcined at 290oC for 3 hours, at 425oC for 3 hours and at 650oC for 8 hours. After this, the catalyst was ground and screened to a 20-35 mesh size. The ground catalyst was given a final calcination at 810oC for 3 hours. Before being evaluated in the microreactor, 8.0g of the calcined catalyst was washed with 100cc of isobutanol.

The foregoing catalysts were tested in ammoxidation runs summarized in the following tables. In these runs the catalyst listed in the left-hand column was in a tubular 3/8 inch I.D. stainless steel fixed bed reactor. Pressure was slightly above atmospheric. The reactor is equipped with a preheat leg and is immersed in a temperature controlled molten salt bath. The feed was fed over the catalyst for at least one-half hour before collection of product; the runs of each example last 30-60 minutes during which the product is collected for analysis.

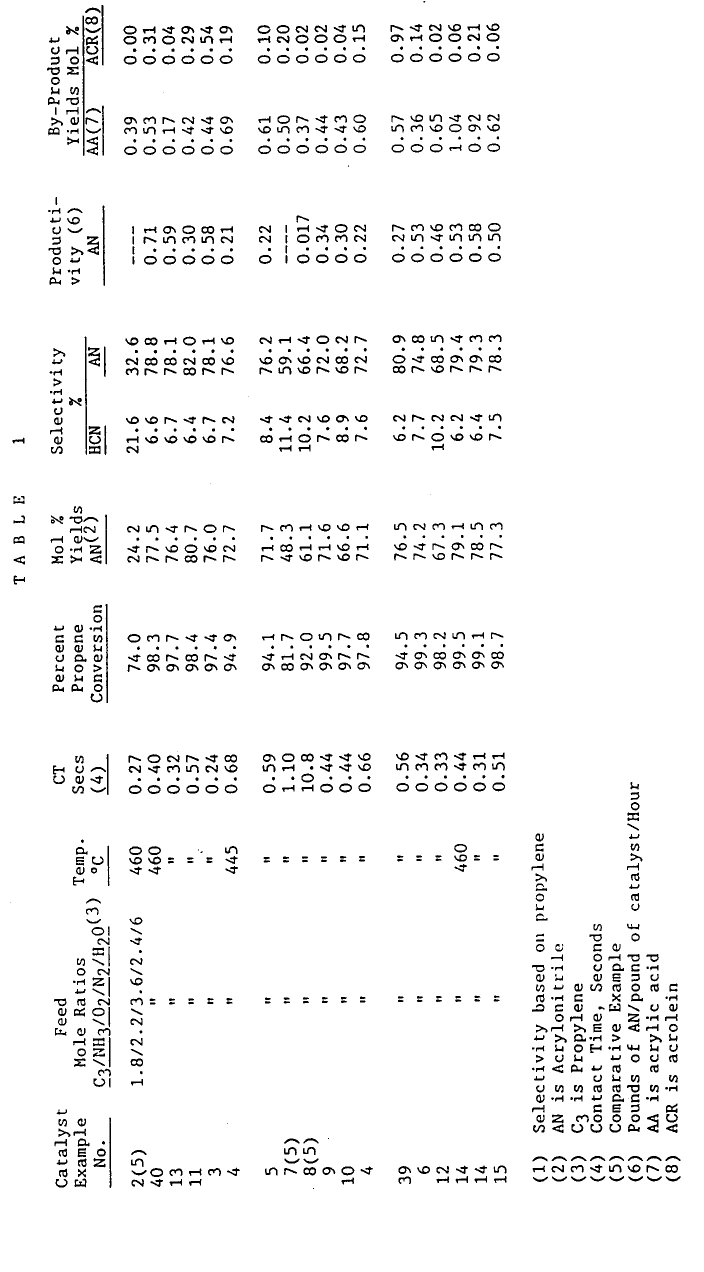

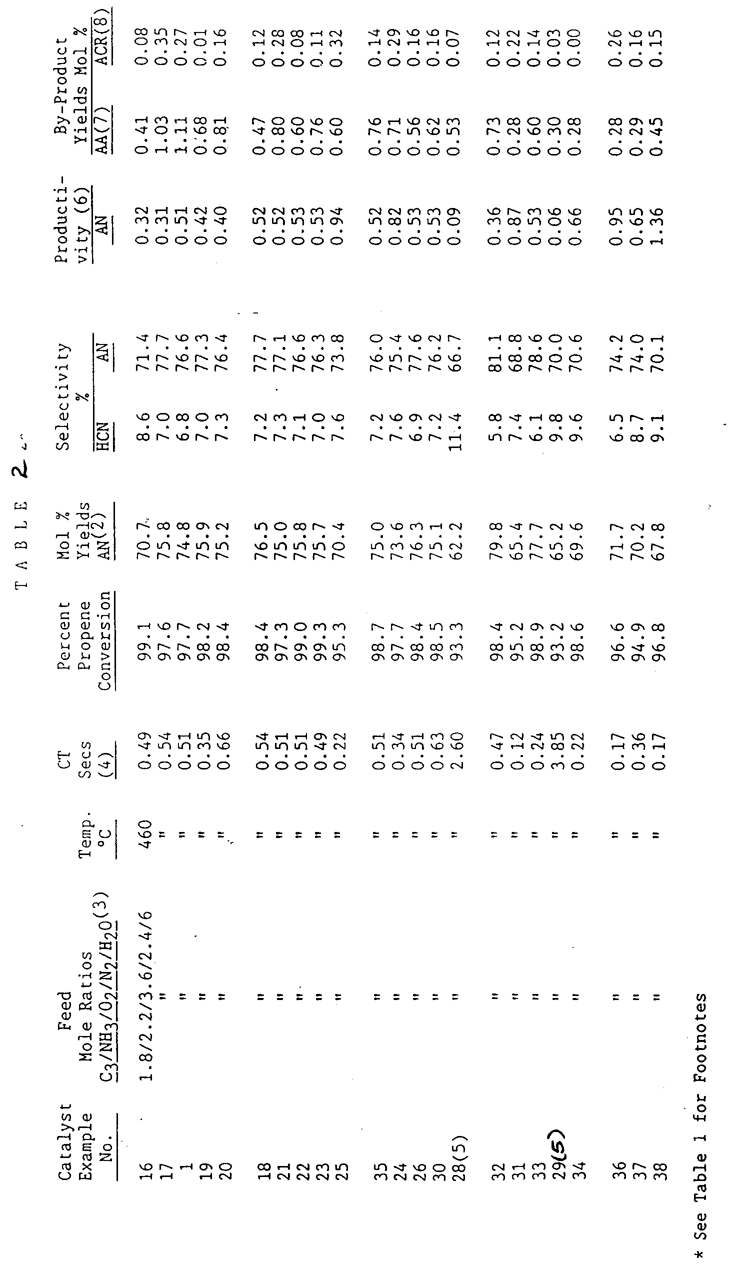

## Claims

1. Method for ammoxidation of C₃ to C₅ mono-olefins to make a,b-mono-unsaturated acyclic nitriles having 3 to 5 carbon atoms and HCN by introducing such mono-olefins, molecular oxygen and ammonia into a reaction zone into vapor phase contact with a solid ammoxidation catalyst, wherein the mol ratio of introduced molecular oxygen and ammonia to said introduced mono-olefin is at least 1.5 and 1.0, respectively, wherein said catalyst contains the elements and proportions indicated by the empirical formula:
V₁SbₐMₘNₙOₓ
where a = 0.5 to 2
M = one or more of: Sn, Ti, Fe and Ga
m = 0.05 to 3
N = one or more of: W, Bi, Mo, Li, Mg, P, Zn, Mn, Te, Ge, Nb, Zr, Cr, Al, Cu, Ce, B
n = 0.0 to 0.5, and
x = number of oxygen atoms required to satisfy the valence requirements of all the remaining elements,
and where the preparation of the catalyst includes firstly contacting in an aqueous dispersion only a vanadium compound and an antimony compound while said vanadium compound is in solution, heating the resulting slurry until the color of the slurry changes from yellow to green to black, adding components M and optionally N and calcining the catalyst at a temperature greater than 750°C.

2. A method of claim 1 wherein a mono-olefin selected from propylene and isobutylene is ammoxidized to acrylonitrile and methacrylonitrile, respectively.

3. A method according to claim 1 or claim 2 wherein m is at least 0.1.

4. A method according to claim 1 or claim 2 wherein m is at most 1.

5. A method according to claim 1 or claim 2 wherein m is in the range from 0.1 to 1.

## Patentansprüche

1. Verfahren zur Ammoxidation von C₃-C₅-Monoolefinen zur Herstellung von a,b-monoungesättigten acyclischen Nitrilen mit 3 bis 5 Kohlenstoffatomen und HCN durch Einführen von solchen Monoolefinen, molekularem Sauerstoff und Ammoniak in eine Reaktionszone in Dampfphasenkontakt mit einem festen Ammoxidationskatalysator, wobei das Molverhältnis von eingeführtem molekularen Sauerstoff und Ammoniak zu dem eingeführten Monoolefin mindestens 1,5 bzw. 1,0 ist, wobei der Katalysator die Elemente und Verhältnisse wie in der empirischen Formel ausgewiesen enthält:
V₁SbₐMₘNₙOₓ
worin a = 0,5 bis 2,
M = eines oder mehrere von: Sn, Ti, Fe und Ga
m = 0,05 bis 3
N = eines oder mehrere von: W, Bi, Mo, Li, Mg, P, Zn, Mn, Te, Ge, Nb, Zr, Cr, Al, Cu, Ce, B
n = 0,0 bis 0,5 und
x = Anzahl der erforderlichen Sauerstoffatome, um den Wertigkeitserfordernissen aller verbleibenden Elemente zu genügen,
und wobei die Herstellung des Katalysators zunächst Inkontaktbringen in einer wässerigen Dispersion nur einer Vanadiumverbindung und einer Antimonverbindung, während die Vanadiumverbindung in Lösung ist, Erwärmen der erhaltenen Aufschlämmung, bis die Farbe der Aufschlämmung sich von gelb zu grün bis schwarz ändert, Zugeben von Komponenten M und gegebenenfalls N und Calcinieren des Katalysators bei einer Temperatur größer als 750°C einschließt.

2. Verfahren nach Anspruch 1, wobei ein Monoolefin, ausgewählt aus Propylen und Isobutylen, zu Acrylnitril bzw. Methacrylnitril ammoxidiert wird.

3. Verfahren nach Anspruch 1 oder Anspruch 2, wobei m mindestens 0,1 ist.

4. Verfahren nach Anspruch 1 oder Anspruch 2, wobei m höchstens 1 ist.

5. Verfahren nach Anspruch 1 oder Anspruch 2, wobei m im Bereich von 0,1 bis 1 liegt.

## Revendications

1. Méthode d'ammoxidation de mono-oléfines C₃ à C₅ pour élaborer des nitriles acycliques a,b-mono-insaturés ayant 3 à 5 atomes de carbone et HCN (cyanure d'hydrogene ou acide prussique) par introduction de ces mono-oléfines, d'oxygène moléculaire et d'ammoniac dans une zone de réaction en contact, en phase vapeur, avec un catalyseur solide d'ammoxidation, méthode selon laquelle le rapport molaire entre l'oxygène moléculaire et l'ammoniac introduits, et lesdites mono-oléfines introduites atteint respectivement 1,5 et 1,0, et selon laquelle ledit catalyseur contient les éléments et proportions indiqués par la formule empirique :
V₁SbₐMₘNₙOₓ
dans laquelle :
a = 0,5 à 2
M = un ou davantage de : Sn, Ti, Fe et Ga
m = 0,05 à 3
N = un ou davantage de : W, Bi, Mo, Li, Mg, P, Zn, Mn, Te, Ge, Nb, Zr, Cr, Al, Cu, Ce, B
n = 0,0 à 0,5, et
x = nombre d'atomes d'oxygène nécessaires pour satisfaire aux exigences de valence de tous les autres éléments,
et selon laquelle la préparation du catalyseur inclut premièrement la mise en contact, dans une dispersion aqueuse seulement, d'un composé de vanadium et d'un composé d'antimoine pendant que ledit composé de vanadium est en solution, le chauffage de la suspension épaisse résultante jusqu'à ce que la couleur de cette suspension passe du jaune au vert, puis au noir, en ajoutant les composants M et, en option, N, et calcination du catalyseur à une température supérieure à 750°C.

2. Une méthode selon la revendication 1, selon laquelle une mono-oléfine sélectionnée à partir de butylène et d'isobutylène est ammoxidisée pour produire respectivement de l'acrylonitrile et du méthacryonitrile.

3. Une méthode selon la revendication 1 ou la revendication 2, selon laquelle m est au minimum 0,1.

4. Une méthode selon la revendication 1 ou la revendication 2, selon laquelle m est au maximum 1.

5. Une méthode selon la revendication 1 ou la revendication 2, selon laquelle m est une valeur comprise entre 0,1 et 1.
